# EUROPEAN PATENT SPECIFICATION

(11) **EP 1 051 616 B1**
(45) Date of publication and mention of the grant of the patent: **27.09.2006**
(21) Application number: 98938365.8
(22) Date of filing: 04.08.1998
(51) Int. Cl.: G01N 33/48

(54) **INTEGRATED COLLECTION AND ASSAY DEVICE FOR SALIVA AND BLOOD**
INTEGRIERTE SAMMEL- UND TESTVORRICHTUNG FÜR SPEICHEL UND BLUT
DISPOSITIF INTEGRE DE PRELEVEMENT ET D'ANALYSE DU SANG ET DE LA SALIVE

(30) Priority: 04.08.1997 US 55254 P
(43) Date of publication of application: 15.11.2000
(73) Proprietor: SEREX, INC., Maywood, NJ 07607 (US)
(72) Inventor: FITZPATRICK, Judith, Tenafly, NJ 07670 (US)
(74) Representative: Miles, John Stephen
(86) International application number: PCT/US1998/016256
(87) International publication number: WO 1999/006827

(56) References cited:
- EP-A- 0 734 686
- EP-A- 0 781 997
- WO-A-94/18891
- WO-A-95/17966
- US-A- 5 116 139
- US-A- 5 500 375

## Description

### Field of the Invention

The present invention is in the field of diagnostic devices, and more particularly in the area of a device for both collection and analysis of a biological fluid such as saliva or blood.

### Background of the Invention

Immunochromatographic assays using a membrane as a solid support in a dipstick or flow through device are established for use in the clinical laboratory as well as for "field" use, *i.e.* use outside of the clinic. Assays using this type of format are available for abused drugs (cocaine, cannabinoid, amphetamine, etc.), pregnancy and fertility testing (hCG and hLH), and infectious diseases (chlamydia, streptoccocus, infectious mononucleosis). These existing chromatographic assays are typically designed and used to analyze blood and urine.

It is preferable in some situations to conduct analysis on a saliva sample rather than on blood or urine. When applicable, saliva testing of certain small analytes has several advantages over blood or urine analysis. Specimen collection is non-invasive and painless. A saliva specimen can be taken at any convenient time, and can be obtained singly or sequentially, if desired, to establish individualized analyte profiles or to adjust drug dosages. Moreover, saliva sampling may be done at any location and can be easily observed, if required. Saliva, unlike plasma or serum, contains few binding proteins and permits measurement of the free or biologically active analytes.

However, saliva also has several attendant problems. To many individuals, saliva is visually repugnant and its handling is undesirable. Saliva may be viscous or foamy; hence it is frequently difficult to dispense or transfer saliva to a receptacle during sampling, particularly with any degree of accuracy. For example, it is difficult to control sample sicking of fresh samples onto a chromatographic membrane. Saliva contains numerous microorganisms and enzymes that can rapidly putrefy the specimen unless stabilized or inhibited with effective preservatives to permit shipment or mailing in existing saliva collection devices. It is difficult and timely to collect more than 0.5 ml of saliva. Saliva generally is processed before use in an immunoassay to remove particulate and soluble mucopolysaccharide constituents. It is difficult to pipette or measure before processing by filtration or centrifugation and a large amount of the specimen may be lost in handling. Specimen processing is obviously costly and time consuming.

Urine is frequently not an acceptable alternative. The urine levels of many analytes do not correlate with blood levels and are subject to variable urine dilution due to patient hydration status and/or tampering, requiring referencing to another analyte, e.g. urinary creatinine. Urine has the additional problem that the sample is usually only collected in private, raising security and handling issues. Blood collection for analysis also presents disadvantages, such as the pain involved in needle sticks and the inherent dangers associated with any use of needles.

Existing devices for collecting and testing of saliva do not fully solve the problems outlined above. There is a need for a device for collection of sample that also serves as the diagnostic device. This is especially a need as diagnostic testing moves from the laboratory to the field where testing will be done by persons not experienced or trained in diagnostic test or sample collection procedures.

### Summary of the Invention

Devices are described herein which combine an integrated diagnostic strip and an attachment for the collection of saliva or the collection of blood. In the preferred embodiment for collection and testing of saliva, the integrated collection and assay device includes a collapsible funnel shaped saliva collector, a saliva processing pad and one or more diagnostic assay strips. The collector, pad, and assay strips are in fluid communication with each other, thereby permitting capillary flow of saliva from the funnel to the assay strips. The device can be used for collection of blood in the same manner as the saliva.

In an embodiment of the device for the collection and analysis of a blood sample which is not part of the present invention, a needle and needle receptacle are attached to the strip to allow collection and direct application of blood to the strip, avoiding any contact, by the party collecting the blood. Assay strips can be incorporated into the collection device, as well as an absorbant pad or filter to remove unwanted tissue or red blood cells from the sample.

### Brief Description of the Drawings

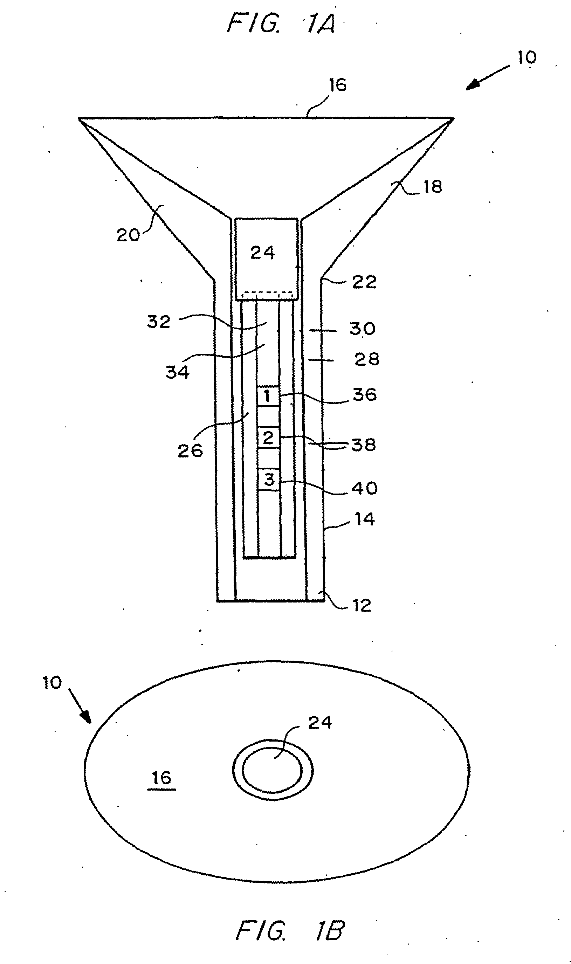

Figures 1a and 1b illustrate a first embodiment of the device for the collection and analysis of a saliva sample. Figure 1a is an elevational view and Figure 1b is a top plan view of the device.

### Detailed Description of the Invention

There is a need for convenient, efficient and cost effective assay devices for the measurement of analytes present in either blood and saliva. The use of such combination devices with saliva would overcome public aversion and fear of drawing blood as well as problems due to undesirable matrix and nonspecific binding effects commonly encountered in the analysis of small.hydrophobic analytes in blood, plasma or serum. The devices disclosed herein overcome the inherent problems with collection of saliva specimens. It is furthermore suitable for both on site interpretation by the patient in the case of simply visual assay systems or for mailing of the completed test to a testing lab for instrumental quantitation and interpretation in the case of more complex assays or test panels requiring evaluation of individualized patient profiles. The same device can therefore be used for a universal home, office, or lab test.

### Saliva Collection Device

Briefly, the integrated collection and assay devices include a fluid collector, a processing and metering pad and/or filter, and one or more assay strips. The fluid collector can be adapted for collection of saliva or blood. The assay strips can be any type presently used. Nitrocellulose is a preferred material. Preferably, the assay strips are laminated dipsticks such as described in U.S. Patent No. 5,500,375.

A preferred embodiment of the device is illustrated in detail in Figures 1a and 1b. The device 10 includes holder 12 having a stem portion 14 and a funnel portion 16. In one embodiment, the holder 12 can be a double sheet of plastic that is laminated together at the stem portion, as described further below. For example, the holder can be made of polyethylene or another clear, flexible plastic. The funnel portion 16 is sealed on the edges to form lateral seals, 18 and 20, that extend down the stem portion 14. Thus, an open top collection "funnel" is formed in the funnel portion 16, which is in fluid communication with the stem portion 14, as shown from the top view in Figure 1b.

At the neck 22 of the device 10. or the juncture of the funnel portion 16 and stem portion 14, is the processing and metering pad 24. Pad 24 is preferably an absorbent pad or sponge which serves to filter oral debris and mucopolysaccharides from the sample. It can be formed of any suitable material, preferably of a fibrous nature, most preferably a material such as a cellulose or cellulose derivative. In some embodiments, the material may be charged or contain substances which effect separations or passage through the filter. For example, the pad may also contain buffers and reagents such as dissociating or mucolytic agents and surfactants which may be required. The pad further serves to meter the amount of sample which is transferred to the assay strip. This is accomplished by selection of the size, shape, porosity and composition of the pad, which can be adjusted as necessary to optimize separation and metering of sample volume.

The device includes one or more assay strips. These may he of the same or different materials, providing for measurement of multiple analytes in the sample. As shown in Fiugre 1a, assay strip 26 extends from the pad 24 into stem portion 14. Assay strip 26 includes carrier 28 which supports thereon membrane 30, which is in liquid communication with pad 24. Assay strip 26 can be any chromatographic assay strip but is preferably designed as described in U.S. Patent Nos. 5.500.375 and 5,710,009. In the preferred embodiment, membrane 30 is a nitrocellulose strip which includes sample application region 32, adjacent to the processing or metering pad 24. The membrane 30 further includes a mobilization zone 34. having immobilized thereupon mobilizable labelled reagent, for example. The membrane also includes one or more trap zones. Three are shown in Figure 1a as 36, 38, 40, which contain various reagents for capturing unreacted analyte or labelled analyte, for example, depending upon the design of the assay. In this preferred embodiment, the membrane further includes one or more detection zones which may be the same as one or more of the trap zones. The section of holder 12 overlying the detection zones is preferably clear so that the results of the assay can be read.

A major advantage of this device is the capability of holding more than one test strip, thus permitting correction for saliva dilution by referencing to another saliva constituent. *e.g.* creatinine, or to another relevant analyte, *e.g.* HDL/LDL ratio. For example, in one embodiment, device 10 includes more than one assay strips in the form of dipsticks, for example, up to about six, where there are three on each side mounted back-to-back.

The holder 12 surrounding the assay strip 26 is preferably laminated to seal the assay strip within the holder in an air tight and substantially liquid tight manner. The collapsible funnel shaped saliva collector, processing pad and one or more laminated dipsticks are all in communication with each other, thereby permitting capillary flow of saliva from the funnel to the detection sites on the dipsticks followed by visual or instrumental detection or quantitation of one or more analytes. An advantage of this device is that the strip can remain funnel up or lying down during and following sample collection. In the latter case, the laminated strip allows the wicking of saliva in the downward position, *i.e.* with the force of gravity. The assay times in these positions are decreased by as much as one half of the time required when the strip is in a vertical position. The strip is also protected in the horizontal (downward) position from flooding because it is laminated or has some other form of seal which controls flow along the surface of the membrane rather than transversely through the pores of the membrane.

In another embodiment, not shown in the Figures, a capillary tube serves as the collection means rather than the funnel of the first embodiment. This allows placement of the collection means directly into the mouth so that saliva can be funneled into the absorbent filtering pad and from thence to the chromatographic assay strip of the device. This embodiment can be constructed shaped either like a conventional thermometer type device or in an inverted "J" shape such that the short end of the "J" is inserted into the mouth and the long arm hangs out of the mouth in an upside down configuration. In this configuration, the assay can be conducted faster because the fluid is moving down hill with the aid of gravity.

Integral to performance of the unitary saliva test device, if it is run in the upside down position, is the ability to limit access of the fluid to the chromatographic region by using the filtering and metering pad.

### Method for Saliva Collection

The device is held vertically to collect a saliva specimen, and the saliva specimen is directly deposited into the funnel. The saliva sample contacts the pad which filters the sample and meters its application to the membrane. More than one dipstick can be accommodated, either side by side or back to back. The flow of saliva into and on the dipstick membrane, and thus the assay time, can be controlled by the porosities of the pad and the dipstick membrane. Lamination of the dipstick membrane further controls the fluid flow and restricts channeling at the sides and above the surfaces of the dipstick membrane.

The assay commences during saliva collection and continues until the test is done. This may require five to ten minutes. The volume of the specimen which will be deposited will typically be between about 0.1 to one ml, depending on the number of dipsticks. After completion of the test, the results may be read visually and interpreted by the user, and the entire device disposed of, without any direct handling of the specimen. Alternatively, for quantitative evaluation or more complex interpretations, the unit may be placed in a sealed container and sent to a testing lab for further analysis, for example, for reading in a reflectometer when in the diagnostic strips include stable reagents such as chromatophors.

## Claims

1. A saliva collection device (10) comprising
a collapsible funnel shaped saliva collector (16),
a saliva processing pad (24), and
one or more diagnostic assay strips (26),
wherein the collector (16), pad (24), and assay strips (26) are in fluid communication with each other, thereby permitting capillary flow of saliva from the funnel (16) to the assay strips (26).

2. The device of claim 1 wherein the device is formed of an integral flexible plastic material.

3. The device of claim 1 wherein the device include diagnostic assay strips for detection or measurement of multiple analytes.

4. The device of claim 1 packaged in a sealed container for transport or disposal.

5. A method for collection and analysis of saliva comprising providing for an individual to spit into a saliva collection device (10) comprising
a collapsible funnel shaped saliva collector (16),
a saliva processing pad (24), and
one or more diagnostic assay strips (26)
wherein the collector (16), pad (24) and assay strips (26) are in fluid communication with each other, thereby permitting capillary flow of saliva from the funnel (16) to the assay strips (26) and collecting the saliva in the device (10) for detection and/or quantitation of one or more analytes in the saliva.

## Patentansprüche

1. Speichelsammelvorrichtung (10), mit
einem kollabierbaren trichterförmigen Speichelkollektor (16),
einem Speichelverarbeitungskissen (24), und
einem oder mehreren Diagnoseteststreifen (26),
wobei der Kollektor (16), das Kissen (24) und die Teststreifen (26) miteinander in Fluidverbindung stehen, wodurch ein Kapillarfluss des Speichels vom Trichter (16) zu den Teststreifen (26) ermöglicht wird.

2. Vorrichtung nach Anspruch 1, wobei die Vorrichtung aus einem integralen flexiblen Kunststoffmaterial geformt ist.

3. Vorrichtung nach Anspruch 1, wobei die Vorrichtung Diagnoseteststreifen zum Erfassen oder Messen von Mehrfach-Analyten aufweist.

4. Vorrichtung nach Anspruch 1, die in einem abgedichteten Behälter für den Transport oder die Entsorgung verpackt ist.

5. Verfahren zum Sammeln und Analysieren von Speichel, bei dem einer Person eine Speichelsammelvorrichtung (10) zur Verfügung gestellt wird, um in diese zu spucken, wobei die Vorrichtung aufweist
einen kollabierbaren trichterförmigen Speichelkollektor (16),
ein Speichelverarbeitungskissen (24), und
einen oder mehrere Diagnoseteststreifen,
wobei der Kollektor (16), das Kissen (24) und die Teststreifen (26) miteinander in Fluidverbindung stehen, wodurch ein Kapillarfluss des Speichels vom Trichter (16) zu den Teststreifen (26) ermöglicht und der Speichel in der Vorrichtung (10) gesammelt wird, um einen oder mehrere Analyte im Speichel zu erfassen und/oder zu quantifizieren.

## Revendications

1. Dispositif de prélèvement de salive (10) comprenant
un récipient de prélèvement de salive en forme d'entonnoir pliable (16),
un tampon de traitement de la salive (24), et
une ou plusieurs bandelettes d'analyse diagnostique (26),
dans lequel le récipient de prélèvement (16), le tampon (24), et les bandelettes d'analyse (26) sont en communication fluide les uns avec les autres, permettant ainsi l'écoulement capillaire de salive depuis l'entonnoir (16) vers les bandelettes d'analyse (26).

2. Dispositif selon la revendication 1 dans lequel le dispositif est formé d'un matériau plastique flexible intégral.

3. Dispositif selon la revendication 1 dans lequel le dispositif comprend des bandelettes d'analyse diagnostique pour la détection ou la mesure d'analytes multiples.

4. Dispositif selon la revendication 1 emballé dans un emballage fermé hermétiquement pour le transport ou la mise à disposition.

5. Procédé de prélèvement et d'analyse de salive comprenant de prévoir qu'un individu crache dans un dispositif de prélèvement de salive (10) comprenant
un récipient de prélèvement de salive en forme d'entonnoir pliable (16),
un tampon de traitement de la salive (24), et
une ou plusieurs bandelettes d'analyse diagnostique (26)
dans lequel le récipient de prélèvement (16), le tampon (24) et les bandelettes d'analyse (26) sont en communication fluide les uns avec les autres, permettant ainsi l'écoulement capillaire de salive depuis l'entonnoir (16) vers les bandelettes d'analyse (26) et le prélèvement de la salive dans le dispositif (10) pour la détection et/ou la quantification d'un ou plusieurs analytes.
